(19) Europäisches Patentamt

European Patent Office

Office européen des brevets

(11) **EP 0 954 325 B1**

(12) **EUROPEAN PATENT SPECIFICATION**

(45) Date of publication and mention
of the grant of the patent:
**11.06.2003 Bulletin 2003/24**

(21) Application number: **95940399.9**

(22) Date of filing: **30.11.1995**

(51) Int Cl.⁷: $A61K\ 35/14$, $A61P\ 37/00$

(86) International application number:
**PCT/IT95/00206**

(87) International publication number:
**WO 96/016666 (06.06.1996 Gazette 1996/26)**

(54) **METHOD FOR THE SPECIFIC IMMUNOADSORPTION OF SELECTED PATHOGENIC FACTORS IN THE TREATMENT OF AIDS**

VERFAHREN ZUR SPEZIFISCHEN IMMUNABSORPTION VON SELEKTIERTEN PATHOGENEN FAKTOREN ZUR BEHANDLUNG VON AIDS

PROCEDE POUR L'IMMUNOADSORPTION SPECIFIQUE DE FACTEURS PATHOGENES SELECTIONNES POUR LE TRAITEMENT DU SIDA

(84) Designated Contracting States:
**AT BE CH DE DK ES FR GB GR IE IT LI LU MC NL PT SE**
Designated Extension States:
**LT LV SI**

(30) Priority: **30.11.1994 IT VR940096**

(43) Date of publication of application:
**10.11.1999 Bulletin 1999/45**

(73) Proprietor: **SANITARIA SCALIGERA S.P.A.**
**37127 Avesa (IT)**

(72) Inventor: **ZUCCATO, Alessandro**
**I-37123 Verona (IT)**

(74) Representative: **Petraz, Gilberto Luigi et al**
**GLP S.r.l.**
**Piazzale Cavedalis 6/2**
**33100 Udine (IT)**

(56) References cited:
| | |
|---|---|
| **EP-A- 0 054 799** | **EP-A- 0 103 184** |
| **EP-A- 0 592 989** | **WO-A-80/02805** |
| **WO-A-88/09670** | **WO-A-92/08734** |
| **WO-A-92/21978** | **WO-A-94/27698** |
| **GB-A- 1 536 425** | **US-A- 4 831 118** |
| **US-A- 5 037 649** | |

## Description

## DEFINITIONS

The following definitions will be adopted throughout the present specification:

## VIRUS HIV:

Any virus belonging to the "Human Immunodeficiency Virus" family;

## THE PATHOGENIC FACTORS:

HIV virus, and/or gp120 antigen, and/or gp120/anti-gp120 immunocomplexes, and/or TNF-$\alpha$, and/or interleukins (IL1$\beta$, IL4, IL6, IL8, IL10), and/or soluble HLA molecules, and/or gp41 protein, and/or immunocomplexes formed by gp41 and anti-gp41 antibodies.

## THE SPECIFIC LIGANDS

Monoclonal and/or polyclonal anti-gp120 antibodies, and/or anti-HLA antibodies, and/or anti-gp41 antibodies, and/or recombinant CD4 molecules, and/or anti-CD4 antibodies, and/or GNA (Galanthus Nivalis Agglutinin) lectin, and/or a peptide fragment of C1q protein and/or native or recombinant C1q protein, and/or Streptococcus G protein, and/or anti-TNF-$\alpha$ antibodies, and/or anti-interleukins antibodies.

## BACKGROUND ART

The present invention relates to use for the preparation of a medical dence for the treatment of Acquired Immunodeficiency Syndrome (AIDS) by a module for carrying out an ex vivo method for the specific immunoadsorption of pathogenic factors.

## TREATMENT OF AIDS

As widely known, HIV virus is a human retrovirus which causes Acquired Immunodeficiency Syndrome (AIDS).

During the acute infection the virus undergoes a massive replication and it is present in patient's peripheral blood (viraemia) for a very short period, followed by a long latency period during which the virus is present in both peripheral and lymphonodal lymphocytes, but is hardly detectable as free virus in peripheral blood. The latency phase is followed by another viraemic phase characterised by the increase of viral particles and antigens in peripheral blood. This phase is typically associated to the onset of Acquired Immunodeficiency Syndrome. HIV virus causes immunodeficiency by integration in CD4 cells and causing their death through both direct (cytotoxical) and indirect mechanisms.

The molecular characteristics of the virus, the dynamics of its integration in the cells, the immune response against it and all the etiopathogenic aspects of the syndrome have constituted the subject-matter of several publications, to which the reader may refer for a complete outline of the state of the art (see, e.g. Fauci A.S. Science 1993, 262:1011-1018).

HIV virus produces some viral antigens which are present in serum or in plasma of HIV-positive patients.

A first antigen is constituted by p24, which is typically present during the primary infection (Sidow M. et al. Br. Med. J. 1988 296:238-40), and which is later hardly detectable, during the syndrome latency phase, due to the production of specific antibodies which mask its recognition by the antibodies which are used for its detection (Lange JMA et al. Br. med. J. 1986 293: 1459-62).

The decline of anti-p24 antibodies in serum, and the simultaneous increase of p24 antigen are associated to progression of the syndrome and to clinical deterioration (Allain JP et al. New. Engl. J. Med. 1987 317: 1124-21).

*In vitro* HIV-infected cells release gp120 protein, which corresponds to the extracellular unit of the viral envelope.

gp120 may be measured in the serum of HIV-positive patients by using an ELISA test, in which an anti-gp120 antibody is bound to the solid phase and the bound antigen is detected by a second enzyme-labelled antibody. The gp120 plasmatic concentration ranges from 1 to 100 ng/ml in AIDS patients (Se-Kyung O. et al. J. AIDS: 251-256, 1992). Furthermore, a fraction of plasma gp120 is complexed with anti-gp120 antibodies. This fraction may be detected by a test which is similar to the preceding one, whereby an antibody specific for human immunoglobulins is used as detector. Use of said test shows that gp120, which circulates in the form of immunocomplexes, is also present before the onset of the Acquired Immunodeficiency Syndrome (SE-Kyung O. et al., J. AIDS: 251-256, 1992).

It has been demonstrated by several laboratories that native or recombinant gp120 has direct immunosuppressive effects, among which: inhibition of T-proliferative responses, sensibilization to lysis of CD4 cells by means of cytotoxic T lymphocytes, direct cytotoxicity on nervous cells, aberrant stimulation of chemotaxis of lymphocytes and monocytes, inference of programmed cell death (apoptosis) (Mann DL et al. J. Immunol. 1987, 138:2640-4; Kornfeld H. et al. Nature 1988 335:445-8; Mittler R.S. et al. Science 1989 245:1380-2; Weinhold KJ et al. J.Immunol. 1989 142:3091-7; Oyaizu N. et al. Proc.Natl. Acad.Sci. USA 1990 87:2379-83; Chirmule N et al. Blood 1990 75:152-9; Brenneman DE et al. Nature 1988 335:639-42; Ameisen J.C. et al. Immunol.Today 1991 12:102-4).

All these effects may result in amplification of T-helper lymphocytes suppression, in destruction of non-infected cells, in neurologic damages and in recruitment of non-infected cells in sites characterised by high virus expression, as spleen of lymphnodes.

All these effects contribute to the pathogenesis of Acquired Immunodeficiency Syndrome. Furthermore, some biological effects of gp120 may be increased or even require the presence of arti-gp120 antibodies cross-linking the protein on the surface of the cells (Mittler R.S. et al. Science 1989: 245:1380-2, CruikShank WW, et al. Biomed. Pharmacother. 1990 44:5-11).

This condition may be detected on most AIDS patients. These immunocomplexes may be pathogenic not only by means of the mechanisms described above, but also through complement activation and a direct proinflammatory effect.

The biological function of gp120 is that of binding to the HIV receptor, the CD4 molecule which is expressed on the membrane of CD4+ helper lymphocytes (Dalgleish G. et al. Nature 1984, 312:763-7; Klatzzann D. et al. Nature 1984, 312: 767-8). After binding to CD4, gp120 is shed from the virus, and the transmembrane protein gp41 mediates the fusion between the virus membrane and the cell membrane.

The HIV virus membrane exhibits, in addition to a pair of envelope proteins (gp120 and gp41), other cellular proteins which are probably passively acquired by the cellular membrane during the budding process of the virus (Arthur L. et al. Science 1992, 258:1935-9). Between the cellular proteins which are present on the virus membrane are the antigens of the major histocompatibility complex (HLA), class II and class I. It is possible to capture HIV by means of anti-HLA antibodies, since said antigens are present on the viral membrane in a quantity which is even higher than the quantity of envelope proteins (Arthur L. et al. Science 1992, 258: 1935-9). Some of these antigens, particularly HLA class I, increase in the serum of AIDS patients during the syndrome (Puppo F. et al. J.Laboratory and Clin.Med. 1991 117:91-100), and they may play a directly immunosuppressive role.

It derives therefrom, that selective displacement from serum or plasma of HIV virus, of its mantle antigens (gp120) and of cellular soluble factors increasing during the syndrome, may give a patient a direct benefit by removing some of the immunodeficiency causes.

The therapies which are currently carried out reduce only partially and temporarily the viral load; however, they cannot reduce all those factors which amplify the immunosuppression (circulating antigens and immunocomplexes).

Considerations similar to those which have been disclosed relative to the HIV virus, gp120 antigen and gp120/antibodies anti-gp120 immunocomplexes may be repeated in what concerns Tumor Necrosis Factor TNF-$\alpha$, some interleukins (IL1$\beta$, IL4, IL6, IL8, IL10), soluble HLA molecules and gp41 protein as well as immunocomplexes formed by gp41 and human anti-gp41 antibodies.

Associated to the progression of Acquired Immunodeficiency Syndrome is an over- or uneven production of TNF-$\alpha$, as well as of other inflammatory cytokynes (e.g. IL1$\beta$, IL4, IL6, IL8, IL10). Hyperproduction of TNF-$\alpha$ induces septic shock, which constitutes a major death cause inside of intensive care units.

A substantial reduction of TNF-$\alpha$ appears to be of basic importance in the septic shock treatment.

Several compositions are known for *in vivo* reduction of TNF-$\alpha$ factor and of other factors cited above (see for instance WO-A1-14464/95, DE-A-4342846, DE-A-4340111, WO-A1-10287/95, EP-A-638556, WO-A2-6077/95, WO-A1-3326/95, EP-A-636369, WO-A1-514/95, WO-A1-27947/94, EP-A-486809, WO-A1-4675/89, US-A-5364930).

Document US-A-5037649 discloses a method for treatment of HIV-infected patients. According to this method, patients suffering form HIV-1 infection are treated by extracorporeal removal of IgG and immune complexes from the patient's blood. This is achieved by withdrawing blood from the patient, separating the blood into its plasma and serum components and contacting the plasma with an immunoadsorbent which is constituted by protein A covalently bound to an inert matrix. However, use of protein A as immunoadsorbent involves several disadvantages and drawbacks, since this immunoadsorbent provides for the adsorption not only of IgG and immune complexes, but also of the patient's antibodies. Removal of antibodies from a patient suffering from immunodeficiency diseases may have highly negative effects on the patient himself.

The latter considerations apply in the case of document WO-A1-88/09670 too, which discloses a method for the extracorporeal removal of antibodies to gp160/120 from an individual infected with HIV.

Document EP-A-0592989 discloses a method for adsorbing tumor necrosis factor TNF and/or lipopolysaccharides LPS from aqueosus liquids by using a chromatography column containing cellulose derivatized with dextrane-sulfate. This document does not disclose use of anti-TNF-$\alpha$-antibodies in a chromatography column in order to selectively remove tumour necrosis factor TNF-$\alpha$-.

Document US-A-4831118 discloses a method for isolating and purifying specific protein from a solution, wherein a protein-specific antibody is attached to an organic substrate matrix to form an antibody-substrate, and the protein to be isolated, in an appropriate buffer solution, is contacted with the antibody-substrate conjugate. This document generally discloses an affinity chromatography process; it does not mention any specific application for removing e.g. interleukins by means of specific anti-interleukins antibodies in AIDS patients. The same applies for the relevant contents of document EP-A-0103184.

## DESCRIPTION OF THE INVENTION

The main purpose of the present invention is the use for the preparation of a medical device for the treatment of Acquired Immunodeficiency Syndrome (AIDS) of a immunopheresis module for immunoadsorption suitable for carrying out an ex vivo method for substracting, according to different cases, the pathogenic factors (i.e. HIV virus, and/or gp120 antigen, and/or gp120/antibodies anti-gp120 immunocomplexes, and/or TNF-α, and/or interleukins (IL1β, IL4, IL6, IL8, IL10), and/or soluble HLA molecules, and/or gp41 protein, and/or immunocomplexes formed by gp41 and human anti-gp41 antibodies) whose presence is increased during a HIV virus infection and which is directly correlated to a state of acquired immunodeficiency.

According to a first form of embodiment of the present invention a module is to be used for the specific immunoadsorption from whole blood or plasma of one or more pathogenic factors selected among HIV virus, gp120 antigen, gp120/anti-gp120 immunocomplexes, TNF-α, interleukins (IL1β, IL4, IL6, IL8, IL10), soluble HLA molecules (class I), gp41 protein, immunocomplexes formed by gp41 and anti-gp41 antibodies, in treatment of Acquired Immunodeficiency Syndrome (AIDS), said module comprising a container including an internal chamber placed between a blood or plasma feeding pipe and an evacuation pipe, as well as a solid phase (11) disposed within said internal chamber, specific ligands being firmly fixed to said solid phase through covalent bindings, each specific ligand being suitable for steadily capturing a corresponding pathogenic factor.

Advantageously, the *ex-vivo* immunoadsorption of pathogenic factors from blood also provides for the separation of the corpuscular part of patient's whole blood from plasma, as well as the adsorption of the pathogenic factors by using the specific ligands in order to obtain plasma featuring a low concentration of pathogenic factors, and mixing such plasma with a suitable quantity of patient's blood corpuscular part.

Immunoadsorption or immunopheresis technique is a specific application of affinity chromatography which is used, in this case, for substracting from plasma and/or blood some specific substances which are directly or indirectly responsible of a given disease.

The module according to the present invention is provided with specific ligands which are inso,lublized on an insoluble solid phase in aqueous solutions, said solid phase being held by a carrier which is permeable to whole blood or plasma.

In the case of treatment of whole blood, venous blood draught from the patient is mixed with an anticoagulant and sent, by means of a peristaltic pump, to a treatment unit containing the specific ligands, the antibodies and the molecules being insolublized, i.e. linked or otherwise fixed to a suitable carrier through covalent bindings, so as to enable the specific substraction of the pathogenic factors by means of a specific immunological antibody-antigen or ligand-receptor reaction.

In the specific case of plasma, venous blood draught from the patient is mixed with an anticoagulant and sent to a cell separator, e.g. of a suitable known type, which carries out a separation of the corpuscular part of blood from plasma.

Plasma is then sent to a treating module or unit which contains a permeable carrier carrying covalently bound insolublized (in the above described sense) specific ligands, thereby enabling a specific substraction of pathogenic factors through a specific immunological antibody-antigen or ligand-receptor reaction.

Plasma which has been treated in this way may be then mixed with a suitable quantity of blood corpuscular part.

According to a further form of embodiment, venous blood collected from a patient and supplemented with an anticoagulant may be preserved within a container, and it undergoes the treatment (i.e. separation of corpuscular part and plasma, adsorption of pathogenic agents and re-mixing with the corpuscular part) only at a second stage (e.g. after several collections, once a predetermined quantity of infected blood has been stored), i.e. before carrying out a massive replacement of patient's blood.

According to the invention, the quantity of specific ligands which are insolublized on a suitable carrier may be sufficient for treating all the patient's blood.

## ILLUSTRATION OF DRAWINGS

Other features and advantages of the present invention will become apparent by reading the following description of some forms of embodiment of the module according to the invention, with the help of the annexed drawings in which:

- Fig. 1 is a schematical block diagram showing an apparatus for collecting blood and substracting pathogenic agents from blood;
- Fig. 2 is a scheinatical illustration of an immunopheresis device for substracting pathogenic agents from extracorporeal plasma comprising a module according to the invention;
- Figures 3 and 4 show, each, a schematical representation of covalent (or of other type) bindings between antibody anti-gp120 and/or HLA molecule of Class I and Class II and/or antibody anti-CD4 and/or recombinant CD4 molecules and a solid carrier constituted by a microchannel inside of a microsphere, as well as of an immunological antigen-antibody reaction;
- Fig. 5 is a schematical section view of a treatment module or unit according to the invention containing specific ligands which are insolublized on a polymeric synthetic matrix;
- Fig. 6 shows a a plan view of a grooved plate belonging to the treatment module of fig. 5;
- Fig. 7 is a section along line IX-IX of fig. 6;
- Fig. 8 is a section view similar to that of fig. 5, showing another form of embodiment of a treatment module according to the invention;

- Fig. 9 shows a plan view, on a reduced scale, of a shell suitable for housing a module according to fig. 8;

- Fig. 10 shows a reversed section view taken along line VI-VI of fig. 9;

- Fig. 11 is a view from below on a reduced scale of another shell suitable for housing a module according to Fig. 8.

## DESCRIPTION OF A FORM OF EMBODIMENT OF THE INVENTION

In the drawings identical or similar parts or components bear the same reference signs.

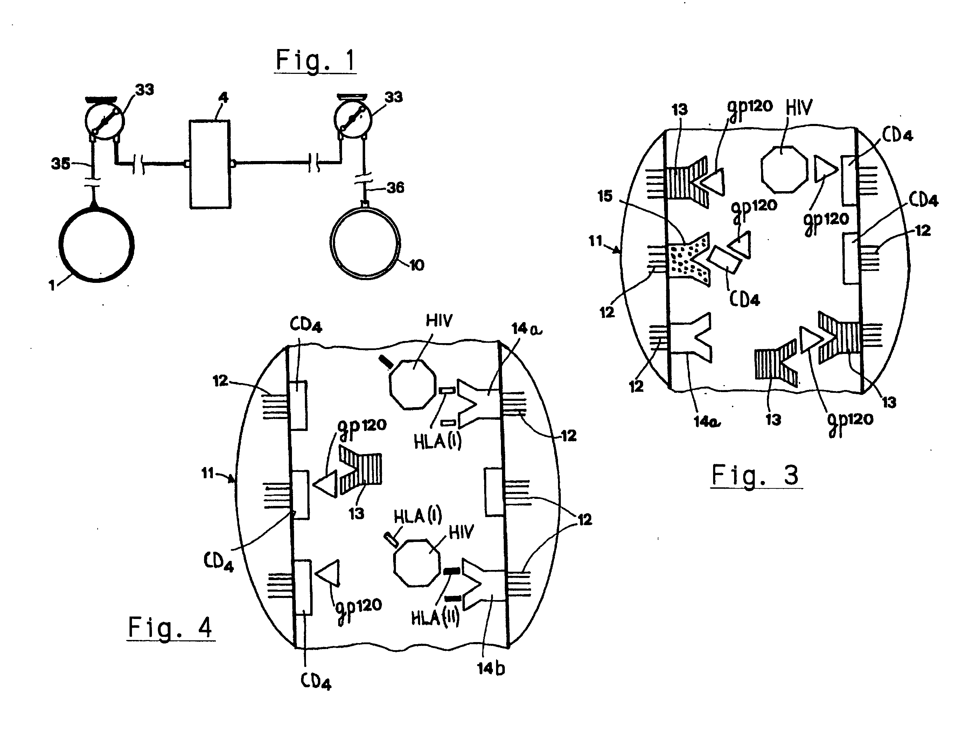

Referring to fig. 1, reference sign 1 generally indicates venous blood draught from a patient infected by HIV virus, reference sign 33 indicates a pair of peristaltic pumps of any suitable known type, one of which being suitable for feeding the patient's blood towards a treatment module or unit 4 through a pipe 35, while the other one feeds the treated blood 10 into a pipe 36.

In figure 2 reference sign 1 indicates venous blood draught from a patient affected with HIV virus, reference sign 2 indicates a centrifugation cell separator, e.g. of any suitable known type, which is able of separating the corpuscular part of blood from plasma, reference sign 3 indicates a pipe for plasma (which contains the pathogenic factors) coming from separator 2 and flowing towards a treatment module or unit 4 containing specific ligands which are insolublized on a solid carrier. The corpuscular part of blood, separated from plasma inside of separator 2, is sent through a pipe 5, to a mixing unit 6.

The plasma treated inside module 4 is also sent through a pipe 7 to mixing unit 6, in order to be mixed with the corpuscular part.

As shown in fig. 2, blood 1 to be treated inside of separator 2, may come from a suitable storing and preserving container 8, inside of which blood 1 might have been stored following to one or more preceding drawings. Furthermore, instead of feeding the plasma treated in the treatment unit or module 4 to mixing unit 6, said plasma may be entirely or partially preserved inside of a suitable storing container 9 before feeding it to mixing unit 6.

This allows a better management of a possible later controlled reinfusion of blood with a low concentration of HIV virus, and/or gp120 antigen, and/or gp120/antibodies anti-gp120 immunocomplexes, and/or TNF-$\alpha$, and/or interleukins (IL1$\beta$, IL4, IL6, IL8, IL10), and/or soluble HLA molecules, and/or gp41 protein, and/or immunocomplexes formed by gp41 and human anti-gp41 antibodies in the patient.

In the example shown in figures 3 and 4 we schematically represented a pipe passing through a microsphere acting as solid phase 11, which is contained inside of treatment unit 4 and to which are firmly fixed through covalent bindings (12) anti-gp120 antibodies (13), and/or anti-HLA of Class I (14a) and/or of Class II (14b) antibodies, and/or anti-CD4 antibodies (15) suitable for acting against HIV virus (which is represented by an octagone), and/or against gp120 antigen (which is represented by a triangle), and or against gp120/antibod anti-gp120 immunocomplexes (represented by a rectangle plus a triangle), and/or soluble HLA molecules of Class I and II (which are represented by a smaller blackened rectangle and by a clear one).

Recombinant CD4 molecules, acting on plasmatic gp120 protein and HIV virus, are also fixed to the solid carrier by means of covalent bindings.

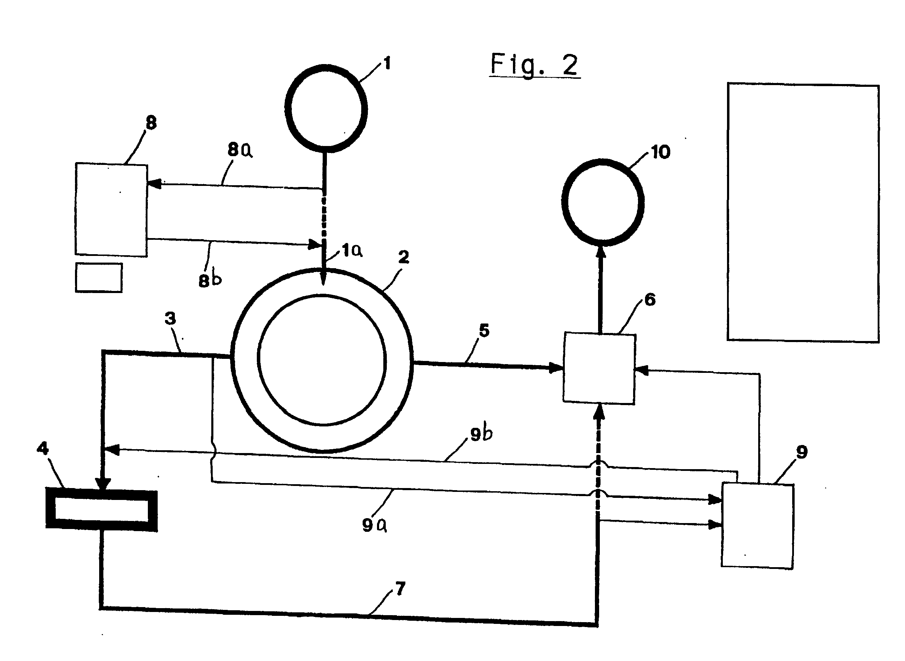

According to a general outline, a treatment unit or module 4 may have the configuration which is schematically illustrated in figures 5 to 11. Module 4 is formed by a container, e.g. formed by a pair of external shells 16, 17, which delimit an internal chamber 18 which, in correspondance of shell 16, is joined to the plasma feeding pipe 3, while on the opposite side, in correspondance of shell 17, is joined to evacuation pipe 7. Chamber 18 houses a mechanical entry septum suitable for distributing in the most uniform way the plasma which flows in, an intermediate solid phase 11, which is constituted, for instance, by microspheres or macroporous modules made of 2-hydroxyethilmethacrylate (HEMA) or agarose, cellulose, glass, polyacrylamide, silica, dextran or other synthetic polymers having a diameter of some tens of microns and bearing monoclonal and polyclonal anti-gp120 antibodies, and/or anti-HLA antibodies, and/or anti-CD4 antibodies and/or recombinant CD4 molecules, and a mechanical exit septum 20, suitable for harvesting the treated plasma and for feeding it into pipe 7.

Solid phase 11 is placed between two microporous filters 21, 22 which are kept: separated from each other by means of a spacing frame 23.

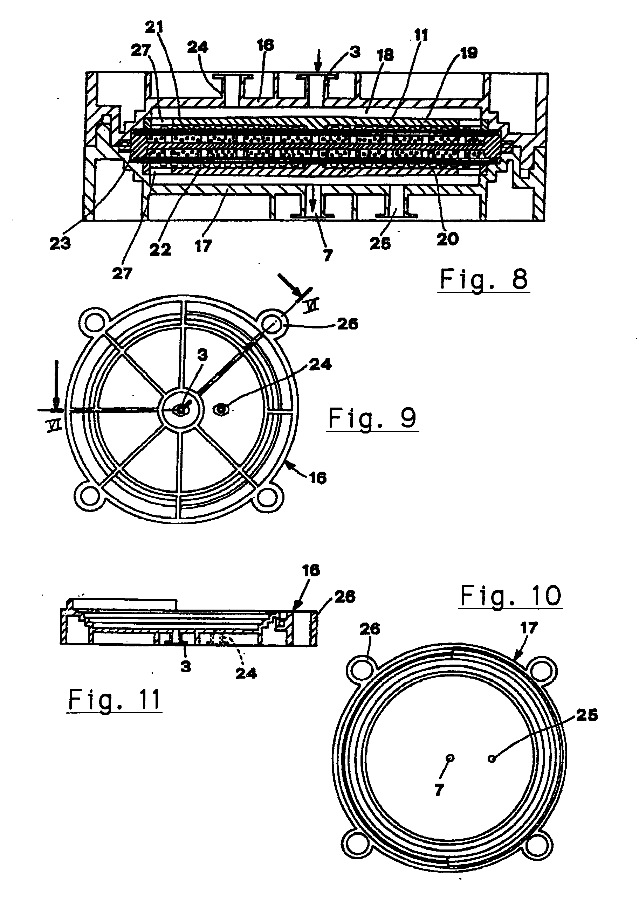

In figure 8 spacing frame 23 extends inwardly between filters 21, 22. Each shell 16, 17 is provided with a respective vent 24, 25 and with peripheral ears 26 cooperating with fastening nuts and bolts.

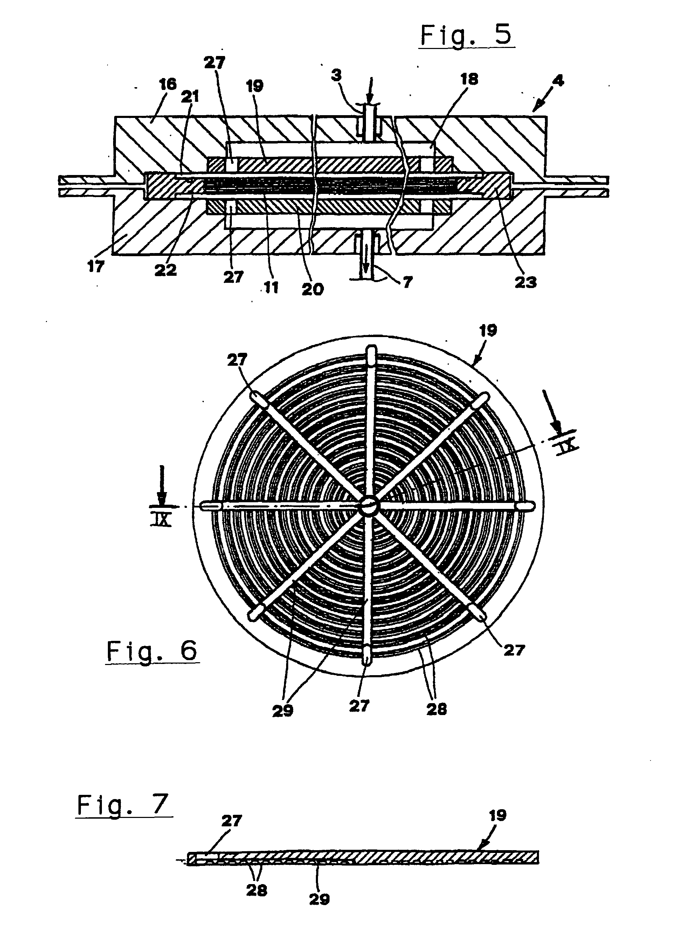

Mechanical septa 19, 20 may have the configuration illustrated in figures 6 and 7, in which the surface directed towards the entry and exit openings is slightly sloping from the centre to the periphery, where some through-holes are provided, while the other surface is either flat or provided with a series of small concentric ribs 28 which are intersected by radial grooves 29 which, taking into consideration entry septum 19, enhance the plasma distribution over the entire surface of filter 21 and which, taking into consideration exit septum 20, enhance the plasma flow towards pipe 7.

As previously mentioned, solid phase 11 may be either constituted by microspheres having a microporous structure or by solid microspheres made of polystyrene or divinylbenzene or ethilen-dimethacrylate having a diameter ranging from 5 to 100 micron (and, more precisely, from 20 to 100 micron for plasma and from 200 to 600 micron for whole blood).

Solid phase 11 may also be constituted by one or more porous membranes, and/or by one or more bundles of capillary vessels made of e.g. polyvinylsulfone or tetrafluorethylene, and/or by spongy or expanded material with open cells.

Anti-gp120 antibodies (13), and/or anti-HLA antibodies of Class I (14a) and/or Class II (14b), and/or recombinant CD4 molecules insolublized on said solid phase 11, come into contact with virus HIV, and/or antigen gp120, and/or immunocomplexes gp120/antibodies anti-gp120, and/or TNF-$\alpha$, and/or interleukins (IL1$\beta$, IL4, IL6, IL8, IL10), and/or soluble HLA molecules, and/or gp41 protein, and/or immunocomplexes formed by gp41 and human anti-gp41 antibodies which are present in plasma and they catch them by binding them by means of an immunological antigen-antibody or ligand-receptor reaction.

Treated plasma flows out of treatment unit 4 exhibiting a lower concentration of virus HIV, and/or antigen gp120, and/or immunocomplexes gp120/antibod anti-gp120, and/or TNF-$\alpha$, and/or interleukins (IL1$\beta$, IL4, IL6, IL8, IL10), and/or soluble HLA molecules, and/or gp41 protein, and/or immunocomplexes formed by gp41 and human anti-gp41 antibodies, and it may then be mixed inside of mixing unit 6 with the corpuscular part of blood which is conveyed therein through pipe 5 and which had previously been separated inside of separator 2, in order to re-build whole blood 10.

The invention has previously been described with reference to an advantageous form of embodiment of the same.

However, it is clear that the invention provides for several forms of embodiment.

As a way of example, the apparatus described above may be used for carrying out a method for substracting TNF-$\alpha$ factor.

In this case solid phase 11, which is represented in fig. 4, may be constituted by a compact, biocompatible and atoxic polymeric resin expressing on its surface active groups suitable for being linked to proteic molecules by means of a covalent binding.

By way of example, a first kind of resin may be bound with a specific ligand as polymyxin B, which is an antibiotic able of binding and desactivating endotoxins produced by gram-negative bacteria; a second kind of resin may be bound with a murine monoclonal antibody (Mab) directed against human cytokin TNF-$\alpha$ or with an either native or recombinant TNF-$\alpha$ receptor.

In the following an example is given of a method for covalently binding proteins or molecules to resin Sepharose-4B manufactured by Pharmacia Biotech (Uppsala, Sweden).

## Method and Materials

0,5 g resin in the form of dry powder are weighed for each 5-8 mg protein, e.g. anti-TNF-$\alpha$ antibody, which have to be bound to the solid carrier (1 g resin yields about 3,5 gel after rehydratation).

After rehydratation by means of 1 mM HCl (5 ml for each gram resin), the resin is washed by means of about 200ml acid for each gram resin.

The dialyzed proteic solution is mixed, inside of a suitable popypropylene container, with the resin washed with HCl (about 2 ml proteic solution for each ml gel).

The mixture formed by gel and proteic solution is stirred for 4 hours at room temperature.

The resin is separated from the buffer solution, which contains the protein which is not covalently bound with the solid carrier, by means of centrifugation at 200-400 g for 10 minutes. The supernatant is harvested by means of aspiration, while the pellet formed by the resin is suspended again with glycine buffer 0,1 M at pH 8,5 (5 ml buffer for each ml resin).

The resin with glycine buffer is stirred for 18 hours at 4°C.

After having separated the resin from the buffer solution by means of centrifugation at 200 g for 10 minutes, the pellet is suspended again with Sodium acetate buffer 0,1 M containing 0,5 M NaCl at pH 4. The resin is washed three times by using the same Sodium acetate buffer. After the third wash the resin is suspended again with physiological Sodium phosphate buffer and it is washed twice by using the same buffer.

After the second wash by means of the phosphate buffer the resin is suspended again with a phosphate buffer containing 0,04% Sodium azyde

## Checking the binding efficiency

The proteic solution harvested after 4 hours incubation with the resin is dialyzed against Sodium carbonate buffer 0,1 M pH 8,5.

The proteic concentration of the solution obtained after a 4 hours incubation with resin Sepharose-4B CNBr and of starting proteic solution are determined by using "Protein Assay" kit of Biorad (Richmond, CA).

The protein quantity which is covalently bound with the resin is determined by using the following formulae:

$$YIELD\% = 100 - (C2)/C1 \times 100$$

where

C1 = proteic concentration before the covalent binding with resin

C2 = supernatant proteic concentration after the first incubation with resin

$$Q = \frac{(C) \times VolP \times YIELD\%}{VolR \times 100}$$

where

Q =      protein quantity (mg) bound to the resin

(C) =    proteic solution concentration before covalent binding with resin

VolP =   proteic solution volume (ml) used for covalent
         binding with resin

VolR =   resin volume (ml) obtained at the end of the
         binding process.

The binding process is then carried out according to the above described method.

**Claims**

1. Use for the preparation of a medical device for the treatment of Acquired Immunodeficiency Syndrome (AIDS) of a module for carrying out an ex vivo method for the specific immunoadsorption from whole blood or plasma of one or more pathogenic factors selected among HIV virus, gp120 antigen, gp120/anti-gp120 immunocomplexes, TNF-α, interleukins (IL1β, IL4, IL6, IL8, IL10), soluble HLA molecules (class I and/or II), gp41 protein, immunocomplexes formed by gp41 and anti-gp41 antibodies, , said module comprising a container including an internal chamber placed between a blood or plasma feeding pipe and an evacuation pipe, as well as a solid phase (11) disposed within said internal chamber, specific ligands being firmly fixed to said solid phase through covalent bindings, each specific ligand being suitable for capturing a corresponding pathogenic factor, **characterised in that** said ligands are constituted by recombinant CD4 molecules, and/or anti-CD4 antibodies, and/or GNA (Galanthus Nivalis Agglutinin) lectin, and/or monoclonal and/or polyclonal anti-gp120, and/or anti-CD4 antibodies, and/or by a peptide fragment of C1q protein and/or native or recombinant C1q protein and/or anti-TNF-α antibodies, and/or anti-interleukins antibodies, and/or anti-HLA (class I and/or II) antibodies, and/or anti-gp41 antibodies.

2. Use according to claim 1, **characterised in that** said specific ligands are steadily bound to an insoluble solid phase in aqueous solutions.

3. Use according to claim 1 or 2, **characterised in that** said solid phase comprises a plurality of microspheres.

4. Use according to claim 3, **characterised in that** said microspheres have a microporous structure.

5. Use according to anyone of claims 3 and 4, **characterised in that** said microspheres are made of a material selected from a group including 2-hydroxyethylmethacrylate, polystyrene, divinylbenzene, polyethylene, acrylamide, polyamide, silica, dextran, agarose, cellulose, glass or other synthetic polymers.

6. Use according to claim 5, **characterised in that** said microspheres have a diameter ranging from 5 to 1000 micron.

7. Use according to claim 2, **characterised in that** said solid phase is constituted by at least a porous membrane.

8. Use according to claim 2, **characterised in that** said solid phase is constituted by at least a bundle of microporous capillary vessels.

9. Use according to claim 8, **characterised in that** the or each bundle of capillary vessels is made of polyvinylsulfone or tetrafluorethylene.

10. Use according to claim 2, **characterised in that** said solid phase is constituted by a spongy or expanded material with open cells.

11. Use according to anyone of the preceding claims, wherein blood is to undergo a treatment for separating the corpuscular part from plasma, wherein only such separated plasma undergoes the adsorption action by means of said specific ligand, so as to obtain plasma exhibiting a low concentration of pathogenic factors, and wherein the plasma to be treated in this way is then mixed with a suitable quantity of the corpuscular part of blood which had previously been separated.

12. Use according to claim 11, **characterised in that** an anticoagulant is to be added to blood before separating the corpuscular part from plasma.

13. Use according to anyone of claims 11 and 12, **characterised in that** said separation into corpuscular part and plasma is to be carried out by centrifugation.

14. Use according to claim 13, **characterised in that** at least part of plasma exhibiting a low concentration of pathogenic agents is to be preserved within a suitable container.

15. Use according to claim 13, **characterised in that** at least part of plasma exhibiting a low concentration of pathogenic agents is to be preserved within a suitable container before mixing it with a suitable quantity of blood corpuscular part.

**Patentansprüche**

1. Verwendung eines Moduls zur Durchführung der spezifischen Immunadsorption aus Vollblut oder einem Plasma eines oder mehrerer pathogener Faktoren ausgewählt aus HIV-Virus, gp120-Antige gp120/Anti-gp120 Immunkomplexen, TNF-$\alpha$, Interleukinen (IL 1$\beta$, IL4, IL6, IL8, IL 10), löslichen HLA-Molekülen (Klasse I und/oder Klasse II), gp41-Protein, aus gp41 und Anti-gp41 Antikörpern gebildeten Immunokomplexen, zur Herstellung einer medizinischen Vorrichtung, zur Behandlung des erworbenen Immunschwächesyndroms (AIDS), wobei das Modul einen Behälter mit einer inneren zwischen einer Blut oder Plasma zuführenden Leitung und einer Entleerungsleitung angeordneten Kammer und einer in der inneren Kammer, angeordneten festen Phase (11) umfasst, wobei an der festen Phase spezifische Liganden mittels kovalenter Bindungen fest angebracht sind, wobei jeder spezifische Ligand dazu geeignet ist, einen korrespondierenden pathogenen Faktor einzufangen, **dadurch gekennzeichnet, dass** die Liganden gebildet sind aus rekombinanten CD4-Molekülen, und/oder Anti-CD4 Antikörpern, und/oder GNA (Galanthus Nivalis Agglutinin) Lektin, und/oder monoklonalen und/oder polyklonalen Anti-gp120, und/ oder Anti-CD4 Antikörpern und/oder durch ein Peptidfragment des C1q-Proteins und/oder nativem oder rekombinantem C1q-Protein und/oder Anti-TNF-$\alpha$ Antikörpern und/oder Anti-Interleukin Antikörpern, und/oder Anti-HLA (Klasse I und/oder Klasse II) Antikörpern, und/oder Anti-gp41 Antikörpern.

2. Verwendung nach Anspruch 1, **dadurch gekennzeichnet, dass** die spezifischen Liganden fest an einer in wässriger Lösung unlöslichen festen Phase gebunden sind.

3. Verwendung nach Anspruch 1 oder 2, **dadurch gekennzeichnet, dass** die feste Phase eine Mehrzahl an Mikrokugeln umfasst.

4. Verwendung nach Anspruch 3, **dadurch gekennzeichnet, dass** die Mikrokugeln eine mikroporöse Struktur aufweisen.

5. Verwendung nach einem der Ansprüche 3 und 4, **dadurch gekennzeichnet, dass** die Mikrokugeln hergestellt sind aus einem Material ausgewählt aus einer Gruppe enthaltend 2-Hydroxyethylmethacrylat, Polystyrol, Divinylbenzol, Polyethylen, Acrylamid, Polyamid, Silika, Dextran, Agarose, Zellulose, Glas oder andere synthetische Polymere.

6. Verwendung nach Anspruch 5, **dadurch gekennzeichnet, dass** die Mikrokugeln einen Durchmesser im Bereich von 5 bis 1000 Mikron aufweisen.

7. Verwendung nach Anspruch 2, **dadurch gekenn-**

**zeichnet, dass** die feste Phase aus wenigstens einer porösen Membran gebildet ist.

8. Verwendung nach Anspruch 2, **dadurch gekennzeichnet, dass** die feste Phase aus wenigstens einem Bündel mikroporöser Kapillargefäße gebildet ist.

9. Verwendung nach Anspruch 8, **dadurch gekennzeichnet, dass** das oder jenes Bündel der Kapillargefäße aus Polyvinylsulfon oder Tetrafluorethylen gebildet ist.

10. Verwendung nach Anspruch 2, **dadurch gekennzeichnet, dass** die feste Phase aus einem schwammartigen oder expandierten Material mit offenen Zellen gebildet ist.

11. Verwendung nach einem der vorhergehenden Ansprüche, wobei das Blut einer Behandlung zur Abtrennung des korpuskularen Teils vom Plasma unterzogen wird, wobei nur derart abgetrenntes Plasma der Adsorption mittels des spezifischen Liganden unterzogen wird, so dass ein Plasma mit einer geringen Konzentration an pathogenen Faktoren erhalten wird, und wobei das derart behandelte Plasma dann mit einer geeigneten Menge des korpuskularen Teils des zuvor abgetrennten Bluts gemischt wird.

12. Verwendung nach Anspruch 11, **dadurch gekennzeichnet, dass** dem Blut vor dem Abtrennen des korpuskularen Teils vom Plasma ein Antikoagulationsmittel zugesetzt wird.

13. Verwendung nach einem der Ansprüche 11 und 12, **dadurch gekennzeichnet, dass** die Trennung zwischen korpuskularem Teil und Plasma durch Zentrifugieren durchgeführt wird.

14. Verwendung nach Anspruch 13, **dadurch gekennzeichnet, dass** wenigstens ein Teil des eine geringe Konzentration an pathogenen Wirkstoffen aufweisenden Plasmas in einem geeigneten Behälter aufbewahrt wird.

15. Verwendung nach Anspruch 13, **dadurch gekennzeichnet, dass** wenigstens ein Teil des eine geringe Konzentration an pathogenen Wirkstoffen aufweisenden Plasmas in einem geeigneten Behälter vor der Mischung mit einer geeigneten Menge an korpuskularem Teil des Blut aufbewahrt wird.

**Revendications**

1. Utilisation pour la préparation d'un dispositif médical destiné au traitement du syndrome d'immunodéficience acquise (SIDA) à l'aide d'un module de réalisation d'une méthode ex vivo d'immunoadsorption, de tout le sang ou plasma, d'un ou de plusieurs facteurs pathogènes sélectionnées parmi le virus VIH et/ou l'antigène gp120 et/ou les immunocomplexes gp120/anti-gp120 et/ou FNT-α et/ou les interleucines (IL1β, IL4, IL6, IL8, IL10) et/ou les molécules HLA solubles (classe I et/ou classe II) et/ou la protéine gp41 et/ou les immunocomplexes formés par les anticorps gp41 et anti-gp41, ledit module comprenant une chambre inteme placée entre un tube d'injection de sang ou de plasma et un tube d'extraction, de même qu'une phase solide (11), à l'intérieur de ladite chambre interne, des ligands spécifiques étant fixés fermement à ladite phase solide par des liaisons covalents, chaque ligand spécifique convenant à la capture constante d'un pathogène correspondant, utilisation **caractérisée en ce que** lesdits ligands sont constitués de molécules CD4 recombinantes eVou d'anticorps anti-CD4 et/ou de lectine GNA (Galanthus Nivalis Agglutinine) et/ou d'anticorps anti-gp120 monoclonaux et/ou polyclonaux et/ou anticorps anti-CD4 et/ou d'un fragment de peptide de protéine Clq et/ou de protéine Clq native ou recombinante et/ou d'anticorps anti-FNT-α et/ou anticorps anti-interieucine et/ou anticorps anti-HLA (classe I et/ou classe II) et/ou anticorps anti-gp41.

2. Utilisation selon la revendication 1, **caractérisée en ce que** lesdits ligands spécifiques sont liés de manière constante à une phase solide insoluble dans des solutions aqueuses.

3. Utilisation selon la revendication 1 ou 2, **caractérisée en ce que** ladite phase solide comprend une pluralité de microsphères.

4. Utilisation selon la revendication 3, **caractérisée en ce que** lesdites microsphères ont une structure microporeuse.

5. Utilisation selon l'une quelconque des revendications 3 et 4, **caractérisée en ce que** lesdites microsphères sont composées d'un matériau sélectionné à partir d'un groupe comprenant le 2-hydroxyéthylméthacrylate, le polystyrène, le divinylbenzène, le polyéthylène, l'acrylamide, le polyamide, la silice, la dextrane, l'agrose, la cellulose, le verre ou d'autres polymères synthétiques.

6. Utilisation selon la revendication 5, **caractérisée en ce que** lesdites microsphères ont un diamètre compris entre 5 et 1000 microns.

7. Utilisation selon la revendication 2 **caractérisée en ce que** ladite phase solide est constituée d'au moins une membrane poreuse.

8. Utilisation selon la revendication 2, **caractérisée en ce que** ladite phase poreuse est constituée d'au moins un faisceau de vaisseaux capillaires microporeux.

9. Utilisation selon la revendication 8, **caractérisée en ce que** le ou chaque faisceau de vaisseaux capillaires est composé de polyvinylsulfone ou de tétrafluoréthylène.

10. Utilisation selon la revendication 2, **caractérisée en ce que** ladite phase solide est composée d'un matériau spongieux ou expansé avec des cellules ouvertes.

11. Utilisation selon l'une quelconque des revendications précédentes, **caractérisée en ce que** le sang doit subir un traitement consistant à séparer la partie corpusculaire du plasma, tandis que ledit plasma séparé subit l'action d'adsorption au moyen dudit ligand spécifique, de façon à obtenir un plasma présentant une faible concentration de facteurs pathogènes, et où le plasma à traiter de cette manière est ensuite mélangé avec une quantité adéquate de la partie corpusculaire de sang qui a été séparée au préalable.

12. Utilisation selon la revendication 11, **caractérisée en ce qu'**un anticoagulant doit être ajouté au sang avant la séparation de la partie corpusculaire du plasma.

13. Utilisation selon l'une quelconque des revendications 11 et 12, **caractérisée en ce que** ladite séparation en partie corpusculaire et en plasma doit s'effectuer par centrifugation.

14. Utilisation selon la revendication 13, **caractérisée en ce qu'**au moins une partie du plasma présentant une faible concentration d'agents pathogènes doit être conservée dans un conteneur adéquat.

15. Utilisation selon la revendication 13, **caractérisée en ce qu'**au moins une partie du plasma présentant une faible concentration d'agents pathogènes doit être conservée dans un conteneur adéquat avant d'être mélangée à une quantité adéquate de partie corpusculaire de sang.

Fig. 1

Fig. 3

Fig. 4

Fig. 2

## Fig. 5

## Fig. 6

## Fig. 7

Fig. 8

Fig. 9

Fig. 10

Fig. 11